# EUROPÄISCHE PATENTSCHRIFT

(11) **EP 2 598 473 B1**
(45) Veröffentlichungstag und Bekanntmachung des Hinweises auf die Patenterteilung: **16.11.2016**
(21) Anmeldenummer: 11743990.1
(22) Anmeldetag: 26.07.2011
(51) Int. Cl.: C07C 201/08, C07C 209/32, C07C 209/36

(54) **VERFAHREN ZUR HERSTELLUNG VON NITROBENZOL DURCH ADIABATE NITRIERUNG**
METHOD FOR PRODUCING NITROBENZENE BY ADIABATIC NITRIDING
PROCÉDÉ DE FABRICATION DE NITROBENZÈNE PAR NITRIFICATION ADIABATIQUE

(30) Priorität: 28.07.2010 DE 102010038519
(43) Veröffentlichungstag der Anmeldung: 05.06.2013
(73) Patentinhaber: Covestro Deutschland AG, 51373 Leverkusen (DE)
(72) Erfinder: KNAUF, Thomas, 41542 Dormagen (DE); RACOES, Alexandre, 47805 Krefeld (DE); RAUSCH, Andreas Karl, 41564 Kaarst (DE); WULF, Dietrich, 50678 Köln (DE)
(74) Vertreter: Levpat
(86) Internationale Anmeldenummer: PCT/EP2011/062827
(87) Internationale Veröffentlichungsnummer: WO 2012/013672

(56) Entgegenhaltungen:
- EP-A1- 1 816 117
- DE-A1-102009 005 324

## Beschreibung

Die Erfindung betrifft ein Verfahren zur kontinuierlichen Herstellung von Nitrobenzol durch Nitrierung von Benzol mit einem Gemisch aus Salpetersäure und Schwefelsäure unter adiabaten Bedingungen, bei dem nicht umgesetztes Benzol aus dem nach Phasentrennung erhaltenen Rohprodukt *vor* dessen Wäsche unter Ausnutzung der adiabaten Reaktionswärme abgetrennt wird.

Die Nitrierung von Benzol mit Salpetersäure zu einem Roh-Nitrobenzol war bereits Gegenstand zahlreicher Veröffentlichungen und Patentanmeldungen. Ein kontinuierliches Verfahren zur Herstellung von Nitrobenzol durch *adiabate* Nitrierung von Benzol durch ein Gemisch von Schwefel- und Salpetersäure (sog. Mischsäure) wurde erstmals in US 2,256,999 beansprucht und ist in heutigen Ausführungsformen in US 4,091,042**,** US 5,313,009 und US 5,763,697 beschrieben.

Den beschriebenen adiabaten Verfahren ist gemein, dass die Ausgangsstoffe Benzol und Salpetersäure in einem großen Überschuss an Schwefelsäure zur Reaktion gebracht werden, welche die frei werdende Reaktionswärme und das bei der Reaktion gebildete Wasser aufnimmt. Charakteristisch für die oben zitierten adiabaten Verfahren ist, dass die in der Reaktionsmischung gespeicherte Reaktionswärme teilweise dazu genutzt wird, die Schwefelsäure, welche durch das gebildete Reaktionswasser und das durch die Salpetersäure eingetragene Wasser verdünnt wurde, wieder aufzukonzentrieren. Die restliche Reaktionswärme verbleibt in der organischen Phase der Reaktionsmischung.

US 5,313,009 beschreibt ein Verfahren zur adiabaten Herstellung von Nitrobenzol, in dem Salpetersäure und Schwefelsäure zur so genannten Mischsäure vermischt werden und in diese Benzol dosiert wird, welches mit der Salpetersäure zu Wasser und im Wesentlichen Nitrobenzol reagiert. Die Temperatur der Reaktionsmischung und die Konzentrationen von Benzol, Salpetersäure und Schwefelsäure werden so gewählt, dass nach einer Reaktionszone eine im Wesentlichen salpetersäurefreie Mischung aus Benzol, Nitrobenzol, Schwefelsäure und Wasser erhalten wird. Benzol wird mindestens in, bezogen auf die Salpetersäuremenge, stöchiometrischen Mengen eingesetzt.

Das im Wesentlichen Salpetersäure freie Reaktionsgemisch, das nach der Reaktionszone erhalten wird, wird einem Phasentrennapparat zugeführt, in dem sich zwei Phasen bilden, eine organische und eine wässrige. Die organische Phase wird als Roh-Nitrobenzol bezeichnet und besteht im Wesentlichen aus Nitrobenzol, Benzol und einer gewissen im Nitrobenzol gelösten Menge an Schwefelsäure und Wasser. Die wässrige Phase wird als Absäure bezeichnet und besteht im Wesentlichen aus Wasser, Schwefelsäure und in der Schwefelsäure gelöstem Nitrobenzol.

Die im Phasentrennapparat abgetrennte Absäure wird in einen Apparat zur Blitzverdampfung des Wassers eingebracht, in dem durch plötzliche Druckerniedrigung und unter Ausnutzung der hohen Temperatur der Absäure, die durch die adiabate Reaktionsführung erreicht wurde, Wasser aus der Absäure verdampft wird, sodass eine aufkonzentrierte Schwefelsäure erhalten wird, deren Konzentration im Wesentlichen der Konzentration vor der Reaktionszone entspricht. Eine über die Schwefelsäureaufkonzentrierung hinausgehende Nutzung der Reaktionswärme wird in US 5,313,009 nicht beschrieben. Daher ist dieses Verfahren nachteilig, weil die freigesetzte Reaktionswärme nicht vollständig genutzt wird.

US 4,091,042 (Spalte 3, Zeilen 44 - 58) beschreibt ein Verfahren zur Herstellung von Nitrobenzol, in dem die Reaktion in vier in Reihe geschalteten gerührten Kesseln durchgeführt wird, wobei die den vierten Rührkessel verlassende Reaktionsmischung in einen kontinuierlich betriebenen Phasentrennapparat geleitet wird, in dem die Absäure von der organischen Phase getrennt wird. Die Absäure wird unter Ausnutzung der in der Absäure gespeicherten Reaktionswärme in einem unter erniedrigtem Druck stehenden Blitzverdampfer aufkonzentriert. Die organische Phase wird kontinuierlich in eine vierstufige Gegenstromextraktionswäsche eingeleitet, wo saure Bestandteile wie Schwefelsäurereste, Dinitrophenol und Pikrinsäure durch den Kontakt mit Natriumcarbonatlösung extrahiert werden. Die gewaschene organische Phase wird einer Wasserdampfdestillation unterzogen, um das überschüssige Benzol zurückzugewinnen. Der Anteil an überschüssigem Benzol im Produkt kann bei diesem Verfahren 0,28 % bis 10,4 % betragen (Siehe Tabelle für die Beispiele 4 bis 9), wobei sich der Benzolüberschuss aus dem Umsatz der Reaktion ergibt und in diesem Verfahren keine technischen Maßnahmen ergriffen werden, den Benzolgehalt im Roh-Nitrobenzol zu reduzieren. Auch dieses Verfahren ist nachteilig, weil die freigesetzte Reaktionswärme nicht vollständig genutzt wird, sondern nur der Anteil, der in der Absäure gespeichert ist.

US 5,763,697 (Spalte 6, Z. 17 - 28) beschreibt ein Verfahren zur Herstellung von Nitrobenzol, in dem das den Rohrreaktor verlassende Nitriergemisch in einem statischen oder auch dynamischen Phasentrennapparat getrennt wird. Die anfallende Absäure wird einer Reinigung und Aufkonzentrierung zugeführt. Das abgetrennte Nitrobenzol mit ca. 5 % bis 10 % an Benzol wird einer Wäsche zum Zwecke der Entfernung von Spuren gelöster Salpetersäure, nitroser Gase und Nitrophenole zugeführt und anschließend in einer Trocknung oder Destillation vom restlichen Benzol und Wasser befreit. Eine Nutzung der in der organischen Phase gespeicherten Energie findet nicht statt, ebenso wenig wie eine technische Maßnahme zur Reduktion des Benzolgehaltes im Nitrobenzol vor der Wäsche.

Es gab aber auch Versuche, überschüssiges Benzol mit Hilfe der Reaktionswärme von der Reaktionsmischung abzutrennen.

US 3,928,475 beschreibt ein Verfahren zur Herstellung von Nitrobenzol bei dem Benzol und Salpetersäure in etwa stöchiometrischen Mengenverhältnissen in ein Nitrobenzol, Benzol und Schwefelsäure enthaltendes Reaktionsgefäß eingeleitet und dort vermischt werden und aus dieser Mischung ein Wasser/Benzol-Azeotrop verdampft wird, wobei die wässrige Phase des Azeotrops Salpetersäure enthält und die organische Phase des Azeotrops neben Benzol auch Nitrobenzol enthält. Dieses Verfahren ist jedoch nachteilig, weil die Reaktionswärme auch zur Verdampfung von Salpetersäure und Nitrobenzol genutzt werden muss. Außerdem kann die in der wässrigen Phase des Azeotrops enthaltende verdünnte Salpetersäure nicht in das Reaktionsgefäß zurückgeführt werden, sondern wird zur Abwasserbehandlung entsorgt.

US 3,981,935 stellt eine auf gleiche Weise nachteilige Ausführungsform des in US 3,928,475 beschriebenen Verfahrens dar. Dort wird die organische Phase des Azeotrops in einem Nachreaktor weiter umgesetzt und nicht in den Hauptreaktor zurückgeführt. Die Nachteile der US 3,928,475 bestehen daher unverändert.

Das Verfahrenskonzept der Nitrobenzolherstellung bei gleichzeitiger azeotroper Destillation wird auch von Hochstrasser und Renken beschrieben (P. Hochstrasser, A. Renken, Chem.-Ing.-Tech. 59 (1987), Nr. 2, S. 172 - 173), wobei dort die Problematik der Salpetersäureverluste im Detail ausgeführt wird. So ist bei der gleichzeitigen Durchführung von Reaktion und Destillation immer mit einem vergleichsweise hohen Salpetersäureverlust zu rechnen, da mit dem verdampften Reaktionswasser Salpetersäure ausgeschleust wird und so zu den Verlusten, die durch eine gewisse Löslichkeit von Salpetersäure in der organischen Phase ohnehin entstehen, eine weitere Verlustquelle hinzukommt.

Das in den Reaktionsapparaten gebildete und von der Säurephase abgetrennte Roh-Nitrobenzol wird gemäß dem Stand der Technik einer Wäsche und einer destillativen Aufarbeitung unterzogen, wie beispielsweise in EP 1 816 117 A1 (Seite 2, Zeile 26 - 42), US,409,1,042 (siehe oben) oder US 5,763,697 (siehe oben) beschrieben. Diese Vorgehensweise bedeutet eine Abkühlung von Roh-Nitrobenzol von der Reaktionsendtemperatur, die üblicherweise zwischen 100 °C und 145 °C liegt, auf weniger als 50 °C für die Waschstufe. Die destillative Abtrennung, die dem Abtrennen des überschüssigen Benzols dient, erfordert eine erneute Aufheizung des gesamten Produktstromes auf die Siedetemperatur von Nitrobenzol, d. h. 211 °C bei Atmosphärendruck, oder entsprechend weniger bei verringertem Druck, z. B. 170 °C bei 350 hPa. Dieses Verfahren ist nachteilig, da der Energieinhalt des Roh-Nitrobenzolstroms nicht vollständig genutzt wird, da das Temperaturniveau niedrig ist, und zusätzlich der Roh-Nitrobenzolstrom nach der Wäsche durch Einsatz zugekaufter Energie aufgeheizt werden muss. Durch das erforderliche erneute Aufheizen nach der Wäsche ist dieses Verfahren aus energetischer Sicht nachteilig, aber auch apparativ aufwändig, da die Destillationskolonne am Ende des Verfahrens so ausgerüstet sein muss, dass der für die Wasserwäsche abgekühlte Nitrobenzolstrom wieder bis zum Siedepunkt aufgeheizt werden kann.

Aufgabe der vorliegenden Erfindung war es daher, ein Verfahren bereitzustellen, das den energetischen Aufwand der Nitrobenzolherstellung verringert, indem es die Ausnutzung der Reaktionswärme optimiert, und es dabei gleichzeitig ermöglicht, apparative Vorteile zu realisieren.

Überraschend wurde gefunden, dass die Aufgabe gelöst werden kann durch ein Verfahren zur kontinuierlichen Herstellung von Nitrobenzol durch
a) Nitrierung von Benzol mit einem Gemisch aus Salpetersäure und Schwefelsäure (Mischsäure) unter adiabaten Bedingungen;
b) Trennung des in Schritt a) erhaltenen Verfahrensproduktes durch Phasentrennung in eine wässrige Phase, welche mindestens Schwefelsäure, Nitrobenzol und Benzol enthält (= Absäure), und in eine organische Phase, welche mindestens Nitrobenzol und Benzol enthält (= Roh-Nitrobenzol);
c) Überführung der in Schritt b) erhaltenen wässrigen Phase in einen Verdampfer, in welchem die Schwefelsäure durch Druckerniedrigung aufkonzentriert wird, wobei dem Verdampfer ein gasförmiger Strom, enthaltend mindestens Wasser, Nitrobenzol und Benzol, entnommen und anschließend kondensiert wird, und wobei die erhaltene aufkonzentrierte Schwefelsäure in Schritt a) zurückgeführt wird;
d) destillative Abtrennung von 20 Massen-% bis 100 Massen-%, bevorzugt von 50 Massen-% bis 100 Massen-%, besonders bevorzugt von 90 Massen-% bis 100 Massen-%, des in der in Schritt b) gewonnenen organischen Phase enthaltenen Benzols durch Verdampfung von Benzol unter Ausnutzung der in Schritt a) angefallenen adiabaten Reaktionswärme in der Verdampfung, wobei ein an Benzol abgereichertes vorgereinigtes Nitrobenzol erhalten wird;
e) Wäsche des in Schritt d) erhaltenen vorgereinigten Nitrobenzols und anschließende Abtrennung von Wasser durch Phasentrennung, wobei gereinigtes Nitrobenzol erhalten wird.

*Unten adiabaten Bedingungen* bedeutet dabei, dass die Reaktion zwischen Benzol und Mischsäure (Schritt a)) nicht thermostatisiert wird. Die Edukte Benzol und Mischsäure werden auf die jeweils gewünschte Temperatur gebracht, vermischt und reagieren dann miteinander, ohne dass die (beträchtliche) Reaktionswärme durch ein Kühlmedium abgeführt wird. Die Reaktionswärme spiegelt sich bei dieser Verfahrensführung quantitativ (mit Ausnahme geringfügiger, unvermeidbarer Wärmeverluste) im Temperaturanstieg der Reaktionsmischung wieder.

Wird die destillative Abtrennung des Benzols wie erfindungsgemäß vorgeschlagen vor der Wäsche ausgeführt, so ergeben sich die folgenden Vorteile:
i) Die im Roh-Nitrobenzol gespeicherte Reaktionswärme kann zur Verdampfung des Benzols in Schritt d) genutzt werden. Somit ist eine Aufheizung, wie sie bei der im Stand der Technik üblichen Destillation nach der Wäsche erforderlich ist, nicht mehr erforderlich, wodurch sich eine deutliche Energieersparnis ergibt (Beispiel 2).
ii) Auf die im Stand der Technik üblichen Destillationskolonne und die Wärmeaustauscher nach der Wäsche kann verzichtet werden, sofern sichergestellt ist, dass das Nitrobenzol auch wassergesättigt seiner weiteren Verwendung zugeführt werden kann. Dies ist zum Beispiel dann der Fall, wenn das Nitrobenzol in einem Verfahren zur Anilinherstellung eingesetzt wird, da bei der Anilinsynthese ohnehin Wasser entsteht, dieses den Prozess also nicht stört, bzw. sogar vorteilhaft sein kann (siehe EP 0 696 573 B1 und EP1 882 681 A1).
iii) Die alternative Benzolabtrennung in Schritt d) hat außerdem den Vorteil, dass die Abtrennung von Benzol unmittelbar nach Reaktion (Schritt a)) und Phasentrennung (Schritt b)) auch die hydraulische Belastung in der Wäsche (Schritt e)) verringert.
iv) Durch die Benzolabtrennung *vor* der Wäsche verkürzen sich die Phasentrennzeiten *in* der Wäsche (Schritt e)), wodurch die Volumina der zur Phasentrennung vorgesehenen Behälter verringert werden können (siehe Beispiel 5).

Durch die Ausnutzung der Reaktionswärme, die in der organischen Phase gespeichert ist, wird das Verfahren zur Herstellung von Nitrobenzol daher nicht nur energetisch, sondern auch apparativ optimiert.

Das erfindungsgemäße Verfahren ist durch eine Reihe von Merkmalen charakterisiert, die sich nicht im Stand der Technik finden. So werden bei der erfindungsgemäßen Herstellung von Nitrobenzol durch adiabate Nitrierung von Benzol mit anschließender Abtrennung von Benzol aus dem Reaktionsgemisch noch vor der Wäsche eine höhere Anlagenverfügbarkeit, niedrigere Instandhaltungskosten, niedrigere Investkosten und geringere Energiekosten erreicht, im Vergleich zu den bisher üblichen Verfahren, bei denen die Abtrennung von Benzol erst im letzten Schritt der Nitrobenzolaufarbeitung erfolgt. Neben den überraschend positiven energetischen und apparativen Effekten ist die erfindungsgemäße Vorgehensweise für den Fachmann auch deshalb nicht naheliegend, weil an die Handhabung des Roh-Nitrobenzols aufgrund von dessen Säuregehalt hohe werkstofftechnische Anforderungen gestellt werden müssen, wodurch zusätzliche Kosten entstehen. Der Fachmann würde gemäß dem Stand der Technik das Benzol aufgrund der Werkstoffproblematik nicht vor der Wäsche abtrennen, weil er einen säurebeständigen und damit vergleichsweise teuren Werkstoff einsetzen müsste, ohne dass ihm aufgrund seines allgemeinen Fachwissens bekannt ist, dass sich diese zusätzlichen Kosten an anderen Stellen im Verfahren wieder amortisieren.

Die Schritte a) und b) des erfindungsgemäßen Verfahrens können nach jedem beliebigen Verfahren zur Herstellung von Nitrobenzol durchgeführt werden, solange es sich in Schritt a) um ein adiabates Verfahren handelt und die Temperatur des in Schritt b) erhaltenen Roh-Nitrobenzols zwischen > 100 °C und 145 °C, bevorzugt zwischen > 120 °C und 140 °C liegt. Bevorzugt wird Schritt a) des erfindungsgemäßen Verfahrens so durchgeführt, wie in DE 10 2008 048713 A1, Absatz [0024] beschrieben. Schritt b) und c) des erfindungsgemäßen Verfahrens sind grundsätzlich aus dem Stand der Technik bekannt. Bevorzugt werden die Schritte b) und c) so durchgeführt, wie in EP 2 070 907 A1, Absätze [0024] und [0027] beschrieben.

In Schritt d) wird das Benzol teilweise bis vollständig in einer thermischen Trennoperation vom Roh-Nitrobenzol abgetrennt, die bevorzugt als Destillationskolonne mit mehreren theoretischen Trennstufen ausgeführt ist. Dabei wird bevorzugt bei vermindertem Druck, und zwar bevorzugt bei absoluten Drücken am Kopf der Destillationskolonne zwischen 0,05 bar und 1,0 bar, besonders bevorzugt zwischen 0,1 bar und 0,5 bar, gearbeitet. Der Zulauf zur Destillationskolonne kann neben dem aus Schritt b) stammenden Roh-Nitrobenzolstrom auch den organischen Strom umfassen, der bei der Schwefelsäureaufkonzentrierung in Schritt c) durch Kondensation des gasförmigen Stroms, enthaltend mindestens Wasser, Nitrobenzol und Benzol, und anschließende Abtrennung des Wassers aus dem Kondensat in einem Phasentrennapparat, erhalten wird. In dieser speziellen Ausführungsform ist demnach der Gegenstand der Erfindung ein Verfahren, bei dem der in Schritt c) erhaltene kondensierte gasförmige Strom, enthaltend mindestens Wasser, Nitrobenzol und Benzol, nach Abtrennung von Wasser der destillativen Abtrennung von Benzol in Schritt d) zugeführt wird.

Der Zulauf zur Destillationskolonne enthält bevorzugt 2,0 Massen-% bis 15 Massen-% an Benzol, bezogen auf die Gesamtmasse des Zulaufs. Darüberhinaus sind im Zulauf zur Destillationskolonne die Nebenprodukte der Benzolnitrierung (insbesondere Dinitrobenzol und Nitrophenole) sowie Wasser und Schwefelsäure vorhanden. Auch Salpetersäure sowie gelöste nitrose Gase können enthalten sein.

Die Destillationskolonne kann beliebig, bevorzugt als Packungs- oder Bodenkolonne ausgeführt sein. Die Destillationskolonne sollte zwischen 3 und 40, bevorzugt zwischen 5 und 20, besonders bevorzugt zwischen 7 und 15 theoretische Trennstufen aufweisen. Die Destillationskolonne kann mit oder ohne Beheizung ausgeführt werden. Ist die vollständige Abtrennung des Benzols angestrebt, so ist eine zusätzliche Beheizung bevorzugt, z. B. durch einen Umlaufverdampfer, da die *vollständige* Benzolabtrennung im Allgemeinen mehr Energie erfordert, als im Roh-Nitrobenzol bei adiabater Reaktionsführung gespeichert ist. Aufgrund der Anwesenheit von gelösten nitrosen Gasen kann es sinnvoll sein, ein Inertgas in die Destillationskolonne einzuspeisen, um dadurch die gasförmigen Stickoxide effektiv ausschleusen zu können.

Das abgetrennte Benzol wird durch ein- oder mehrstufige Kondensation verflüssigt und einem Phasentrennapparat zugeführt, um gleichfalls kondensiertes Wasser abzutrennen. Das abgetrennte Benzol wird bevorzugt in die Reaktion (Schritt a)) zurückgeführt. Dazu sollte es weniger als 10 Massen-% Nitrobenzol, bevorzugt weniger als 4,0 Massen-% Nitrobenzol, jeweils bezogen auf die Gesamtmasse des in die Reaktion (Schritt a)) zurückzuführenden benzolhaltigen Stromes, besonders bevorzugt gar kein Nitrobenzol enthalten.

Soll das in Schritt b) erhaltene Roh-Nitrobenzol *möglichst vollständig* von Benzol befreit werden, so wird die Destillation in Schritt d) so ausgelegt (bspw. durch eine entsprechend hohe Anzahl theoretischer Trennstufen), dass die angestrebten Restbenzolgehalte erreicht werden. Gegenstand dieser Ausführungsform der Erfindung ist dabei insbesondere ein Verfahren, bei dem in Schritt d) > 99,80 Massen-% b is 100 Massen-%, bevorzugt > 99,95 Massen-% bis 99,99 Massen-%, besonders bevorzugt > 99,99 Massen-% bis 99,999 Massen-% des in der in Schritt b) gewonnenen organischen Phase enthaltenen Benzols destillativ abgetrennt werden. Die 100%ige Abtrennung des Benzols kann technisch sehr aufwändig sein, sodass in den bevorzugten Ausführungsformen eine kleine Restmenge (bevorzugt 100 ppm, besonders bevorzugt 10 ppm) des Benzols nicht abgetrennt wird.

In vielen Fällen kann das in Schritt e) gewonnene gereinigte Nitrobenzol ohne weitere Reinigungs- oder Trocknungsschritte weiter verwendet werden. Gegenstand dieser Ausführungsform der Erfindung ist demnach ein Verfahren, bei dem das in Schritt e) gewonnene gereinigte Nitrobenzol nicht weiter aufgereinigt wird und direkt weiteren Verwendungen zugeführt wird. Bevorzugt ist die Verwendung eines gereinigten Nitrobenzols erhalten durch das erfindungsgemäße Verfahren gemäß den Schritten a) bis e) in der Hydrierung zu Anilin.

Es ist auch möglich, Benzol in Schritt d) nur teilweise abzutrennen. Gegenstand dieser Ausführungsform der Erfindung ist ein Verfahren, bei dem in Schritt d) 20 Massen-% bis 99,8 Massen-%, bevorzugt 50 Massen-% bis 95 Massen-%, besonders bevorzugt 70 Massen-% bis 85 Massen-%, des in der in Schritt b) gewonnenen organischen Phase enthaltenen Benzols abgetrennt werden. Dieses Vorgehen ist auch insofern vorteilhaft, als bereits eine teilweise Abtrennung des Benzols die Phasentrennzeit in der Wäsche reduziert. In einer bevorzugten Ausführungsform der nur teilweisen Abtrennung des Benzols vor der Wäsche schließt sich eine destillative Abtrennung des restlichen Benzols und vorhandenen Wassers aus dem gereinigten Nitrobenzol an. Gegenstand dieser bevorzugten Ausführungsform der Erfindung ist demnach ein Verfahren, bei dem in Schritt d) 20 Massen-% bis 99,8 Massen-%, bevorzugt 50 Massen-% bis 95 Massen-%, besonders bevorzugt 70 Massen-% bis 85 Massen-%, des in der in Schritt b) gewonnenen organischen Phase enthaltenen Benzols abgetrennt werden und sich an Schritt e) anschließt:
f) Destillative Abtrennung von Benzol und Wasser aus dem in Schritt e) erhaltenen gereinigtem Nitrobenzol, wobei getrocknetes Rein-Nitrobenzol erhalten wird.

Ob die Durchführung des erfindungsgemäßen Verfahrens gemäß den Schritten a) bis e) oder gemäß den Schritten a) bis f) vorteilhaft ist, hängt zum einen vom Verwendungszweck des Nitrobenzols ab. Verwendungen, für die trockenes Nitrobenzol unabdingbar ist, machen die Durchführung des erfindungsgemäßen Verfahrens gemäß den Schritten a) bis f) erforderlich, während bei Verwendungen von Nitrobenzol, in denen Wasser nicht stört, prinzipiell beide Ausführungsformen einsetzbar sind. Welche Ausführungsform in letzterem Fall vorteilhaft ist, hängt dann meist von den gegebenen Randbedingungen einer Produktionsanlage ab. Wird bspw. eine Nitrobenzolanlage neu gebaut, kann es vorteilhaft sein, auf Schritt f) zu verzichten, weil damit ein Destillationsapparat eingespart werden kann. Soll das erfindungsgemäße Verfahren in einer bestehenden Produktionsanlage integriert werden, die bereits über einen für Schritt f) geeigneten Destillationsapparat verfügt, kann die Ausführungsform gemäß den Schritten a) bis f) sinnvoller sein. Es ist daher durchaus vorstellbar, dass bei gleichen Verwendungszwecken des Nitrobenzols und damit gleichen Reinheitskriterien in einem Fall die Ausführungsform bestehend aus den Schritten a) bis e) und in einem anderen Fall die Ausführungsform bestehend aus den Schritten a) bis f) vorteilhaft ist.

Ist die Durchführung des erfindungsgemäßen Verfahrens gemäß den Schritten a) bis f) vorteilhaft, so ist es im Allgemeinen nicht erforderlich, an Schritt f) noch weitere Reinigungsschritte, (wie eine Destillation des Nitrobenzols selbst), anzuschließen. Gegenstand dieser Ausführungsform der Erfindung ist demnach ein Verfahren, bei dem das in Schritt f) gewonnene getrocknete Rein-Nitrobenzol nicht weiter aufgereinigt wird und direkt weiteren Verwendungen zugeführt wird. Bevorzugt ist die Verwendung eines getrockneten Rein-Nitrobenzols erhalten durch das erfindungsgemäße Verfahren gemäß den Schritten a) bis f) in der Hydrierung zu Anilin.

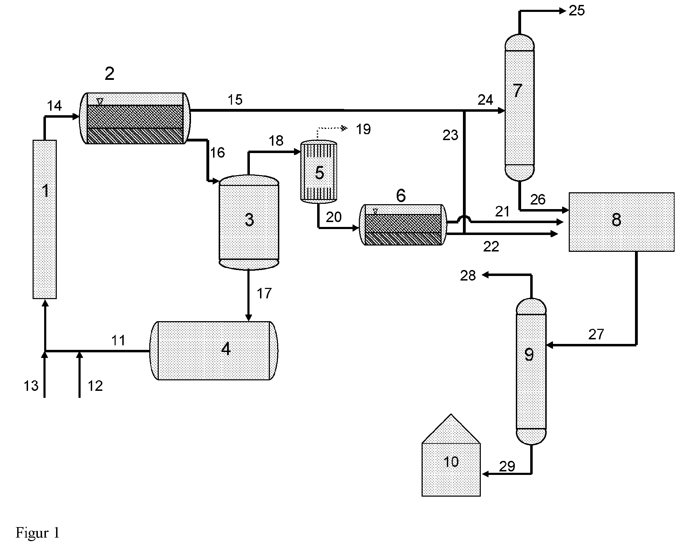

Eine bevorzugte Ausführungsform des Verfahrens zeigt Figur 1. Einem Reaktor (1) werden ein Schwefelsäure- (11), ein Salpetersäure- (12) und ein Benzolstrom (13) zugeführt. Nach vollständiger Umsetzung der Salpetersäure mit dem Benzol zu Nitrobenzol unter adiabter Reaktionsführung wird das nunmehr ca. 130 °C heiße Reaktionsprodukt (14) einem Phasentrennapparat (2) zugeführt, in dem das Reaktionsprodukt (14) in eine organische Phase ((15), = Roh-Nitrobenzol, enthaltend neben Nitrobenzol noch Benzol) und eine wässrige Phase ((16), = Absäure, enthaltend neben Schwefelsäure noch geringe Anteile an Nitrobenzol und Benzol) zerfällt. Die wässrige, hauptsächlich Schwefelsäure umfassende Phase (16) im Verdampfer (3) durch plötzliche Druckerniedrigung (auf 60 mbar bis 120 mbar; (19) = in Richtung der Vakuum-Pumpe) einer Blitzverdampfung von Wasser unterzogen und auf diese Weise aufkonzentriert. Die aufkonzentrierte Schwefelssäure (17) wird im Schwefelsäuretank (4) zur erneuten Verwendung gelagert. Bei der Aufkonzentrierung der Schwefelsäure wird ein Dampfstrom (18) enthaltend Wasser, Schwefelsäure, Nitrobenzol und Benzol erhalten, der in einem Kondensator (5) kondensiert wird. Das verflüssigte Kondensat (20) wird in dem Phasentrennapparat (6) in eine organische und eine wässrige Phase getrennt und die wässrige Phase (21) der Wäsche zugeführt, während die organische Phase entweder ebenfalls der Wäsche (22) oder - bevorzugt - mit dem Roh-Nitrobenzol (15) über (23) zu Strom (24) vereinigt wird. Strom (24) - bestehend aus dem Roh-Nitrobenzol aus dem Phasentrennapparat (15) und gegebenenfalls aus Strom (23) - wird einer Apparatur zur Benzolabtrennung (7) zugeführt, in der Benzol und Wasser über Kopf abgetrennt werden (25) und ein an Benzol abgereichertes vorgereinigtes Nitrobenzol als Sumpfprodukt (26) erhalten wird, das ganz oder teilweise von Benzol befreit wurde. Das vorgereinigte Nitrobenzol (26) wird der Wäsche (8) zugeführt. Der so erhaltene von Nitrophenolen und Salzen weitgehend befreite Strom des gereinigten Nitrobenzols (27) kann optional wieder aufgeheizt und in einer Destillationskolonne (9) von Wasser und gegebenenfalls noch vorhandenem Benzol, welche beide über Kopf abgetrennt werden (28), befreit werden, wodurch getrocknetes Rein-Nitrobenzol (29) erhalten und in Tank (10) gelagert wird.

Die Wäsche des vorgereinigten Nitrobenzols erfolgt bevorzugt als mindestens dreistufige Extraktion, die bevorzugt als Gegenstromextraktion ausgeführt wird. Die Wäsche erfolgt bevorzugt in einem Temperaturbereich von 20 °C bis 95 °C.

Dabei wird in der ersten Stufe durch Kontakt mit Wasser überschüssige Schwefelsäure abgetrennt (sog. saure Wäsche), in der zweiten Stufe werden durch Kontakt mit einem alkalischen Waschwasser organische Nebenprodukte extrahiert (sog. alkalische Wäsche), und schließlich werden in der dritten Stufe (sog. neutrale Wäsche) überschüssige Lauge und Salze abgetrennt. Gegenstand dieser Ausführungsform der Erfindung ist demnach ein Verfahren, bei dem die Wäsche in Schritt e) aus mindestens jeweils einer sauren, alkalischen und neutralen Waschstufe besteht.

Auf die saure Wäsche kann verzichtet werden, wodurch sich jedoch der Bedarf an Lauge in der alkalischen Wäsche erhöht. Geeignete Laugen sind Ammoniakwasser, Alkalicarbonate und - hydrogencarbonate, sowie Alkali- und Erdalkalihydroxide.

Die neutrale Wäsche kann als einstufige oder mehrstufige Wäsche mit Wasser ausgeführt werden. Die neutrale Wäsche kann auch ersetzt werden, indem Membranmodule oder Zentrifugen zur Abtrennung der Laugenreste und Salze eingesetzt werden. Auf die neutrale Wäsche kann auch vollständig verzichtet werden, wenn bereits vorher eine Nitrobenzolqualität erhalten wurde, die den Kundenanforderungen genügt. So sollte zum Beispiel die Konzentrationen an Nitrophenolen am Ende der Wäsche kleiner 200 ppm, bevorzugt kleiner 20 ppm, besonders bevorzugt kleiner 5 ppm sein.

### Beispiele

Die nachfolgenden Beispiele 1 bis 4 beruhen auf Aspen-Simulationen, die an einer bestehenden Nitrobenzolanlage validiert wurden. Die grundsätzliche Verfahrensführung kann Figur 1 entnommen werden, wobei je nach Beispiel nicht alle Apparate eingesetzt wurden.

### Beispiel 1 (Vergleichsbeispiel): Destillative Abtrennung des Benzols erst nach der Wäsche

In diesem Beispiel wurde das Verfahren ohne Apparat (7) simuliert. Daher gelangen die Ströme (15), (21) und (22) direkt in die Wäsche (8). Strom (27) muss von 40 °C auf 170 °C aufgeheizt werden, um in Kolonne (9) Benzol bei 350 mbar bis auf einen Gehalt von 100 ppm abzutrennen. Der Energiebedarf zum vollständigen Abtrennen des Benzols in Kolonne (9) entspricht dem zum Aufheizen des Stroms (27) erforderlichen und beträgt 39,2 kW/t_{Nitrobenzol}.

### Beispiel 2 (erfindungsgemäß): Teilweise destillative Abtrennung des Benzols nur aus Strom (15) vor der Wäsche

In diesem Beispiel wurde das Verfahren mit Apparat (7) als Destillationskolonne mit 7 theoretischen Trennstufen und Verdampfer simuliert. Der Zulauf (24) besteht nur aus dem 127 °C heißen Roh-Nitrobenzolstrom (15), welcher 7 Massen-% Benzol enthält. Die Ströme (21) und (22) gehen somit direkt in die Wäsche (8). Die Kolonne (7) wird bei 350 mbar so betrieben, dass der Benzolgehalt in Strom (26) nur noch 2 Massen-% Benzol beträgt. Dafür sind 15,5 kW/t_{Nitrobenzol} erforderlich. Da jedoch Strom (26) eine Austrittstemperatur von 146 °C aufweist, können 7,5 kW/t_{Nitrobenzol} durch Energieintegration zur Erzeugung von 1,5 bar Dampf wieder gewonnen werden. Da ein Teil des Benzols nun bereits abgetrennt ist, muss Kolonne (9) nur noch mit einer Energiezufuhr von 29,4 kW/t_{Nitrobenzol} betrieben werden, um den Benzolgehalt auf 100 ppm zu verringern. Damit beträgt der gesamte Energiebedarf zum Abtrennen des Benzols in den Kolonnen (7) und (9) unter Berücksichtigung der Energieintegration an Kolonne (7) insgesamt nur 37,4 kW/t_{Nitrobenzol}.

### Beispiel 3 (erfindungsgemäß): Teilweise destillative Abtrennung des Benzols aus den vereinigten Strömen (15) und (23) vor der Wäsche

In diesem Beispiel wurde das Verfahren mit Apparat (7) als Destillationskolonne mit 6 theoretischen Trennstufen und Verdampfer simuliert. Der Zulauf (24) besteht einerseits aus dem 127 °C heißen Roh-Nitrobenzolstrom (15), der 7 Massen-% Benzol enthält, und andererseits aus dem 40 °C warmen Kondensatstrom (23), der ebenfalls 7 Massen-% Benzol enthält. Es ergibt sich für den Kolonnenzulauf (24) eine Mischtemperatur von 110 °C. Die Kolonne (7) wird bei 150 mbar so betrieben, dass der Benzolgehalt in Strom (26) nur noch 2 Massen-% Benzol beträgt. Dafür sind 13,2 kW/t_{Nitrobenzol} erforderlich. Da aufgrund des niedrigen Druckes von 150 mbar, der Strom (26) eine Austrittstemperatur von nur 115 °C aufweist, wird für diesen Fall keine Energieintegration berücksichtigt. Da ein Teil des Benzols nun bereits abgetrennt ist, muss Kolonne (9) nur noch mit einer Energiezufuhr von 23,6 kW/t_{Nitrobenzol} betrieben werden, um den Benzolgehalt auf 100 ppm zu verringern. Damit beträgt der gesamte Energiebedarf zum Abtrennen des Benzols in den Kolonnen (7) und (9) insgesamt nur 36,8 kW/t_{Nitrobenzol}.

### Beispiel 4 (erfindungsgemäß): Vollständige destillative Abtrennung des Benzols aus den vereinigten Strömen (15) und (23) vor der Wäsche

In diesem Beispiel wurde das Verfahren mit Apparat (7) als Destillationskolonne mit 10 theoretischen Trennstufen und Verdampfer simuliert. Der Zulauf (24) besteht einerseits aus dem 127 °C heißen Roh-Nitrobenzolstrom (15), der 7 Massen-% Benzol enthält, und andererseits aus dem 40 °C warmen Kondensatstrom (23), der ebenfalls 7 Massen-% Benzol enthält. Es ergibt sich für den Kolonnenzulauf (24) eine Mischtemperatur von 115 °C. Die Kolonne (7) wird bei 350 mbar so betrieben, dass der Benzolgehalt in Strom (26) nur noch 100 ppm beträgt. Dafür ist am Verdampfer der Kolonne (7) eine Heizleistung von 41,2 kW/t_{Nitrobenzol} erforderlich. Dajedoch Strom (26) eine Austrittstemperatur von 170 °C aufweist, können 22,3 kW/t_{Nitrobenzol} durch Energieintegration zur Erzeugung von 1,5 bar Dampf wiedergewonnen werden. Da das Benzol bereits vollständig aus dem Nitrobenzol entfernt wurde, wird auf eine Destillation nach der Wäsche verzichtet und Kolonne (9) entfällt vollständig. Damit beträgt der gesamte Energiebedarf zum Abtrennen des Benzols in Kolonne (7) unter Berücksichtigung der Energieintegration insgesamt nur 18,9 kW/t_{Nitrobenzol}.

**Tabelle 1: Eckdaten der Beispiele 1 bis 4.**

| **Beispiel:** | **1** | **2** | **3** | **4** |
|---|---|---|---|---|
| Ausführungsform der Benzolabtrennung | Vollständige Abtrennung nach Wäsche (konventionell) | Teilweise Abtrennung vor Wäsche **ohne** Strom (23) | Teilweise Abtrennung vor Wäsche **mit** Strom (23) | **Vollständige** Abtrennung vor Wäsche **mit** Strom (23) |
| Anzahl Trennstufen in Kolonne (7) | n. a. | 7 | 6 | 10 |
| Druckniveau in Kolonne (7) (mbar) | n. a. | 350 | 150 | 350 |
| Druckniveau in Kolonne (9) (mbar) | 350 | 350 | 350 | n. a. |
| Benzolgehalt im Kolonnenzulauf (24) (Massen-%) | n. a. | 7 | 7 | 7 |
| Benzolgehalt im Kolonnen-ablauf (26) (Massen-%) | 7 | 2 | 2 | 0,01 |
| Restbenzol im Rein-Nitrobenzol (29) (ppm) | 100 | 100 | 100 | 100 |
| Spezifischer Energiebedarf (kW/t_{Nitrobenzol}) | 39,2 | 37,4 | 36,8 | 18,9 |

| | | | | |
|---|---|---|---|---|
| n. a.: nicht anwendbar, da Apparat nicht Bestandteil der Simulation war | | | | |

### Beispiel 5 (Zur Illustration des Zusammenhanges zwischen Phasentrennung und Benzolgehalt)

Zur Untersuchung des Einflusses des Benzolgehaltes auf die Phasentrennung wurde Nitrobenzol mit Benzolgehalten zwischen 0 Massen-% und 9 Massen-%, jeweils bezogen auf die Gesamtmasse der Mischung, eingesetzt. Dabei enthielt das Nitrobenzol im Falle a) 2000 ppm an Nitrophenolen und Reste an Schwefelsäure und war im Falle b) frei von Nitrophenolen und Säure. Damit wurde also bei den Proben (a) der Fall einer sauren Wäsche nachgestellt, und im Falle b) die neutrale Wäsche. Für beide Fälle wurde durch die Variation des Benzolgehaltes geprüft, ob sich eine vorherige Benzolabtrennung in der Wäsche bemerkbar macht. In einem 2-Liter-Rührbehälter wurden je 1 Liter der Nitrobenzol-Probe mit jeweils 360 ml an destilliertem Wasser für 1 Minute mit 500 U/min mit einem Scheibenrührer gerührt. Nach Abstellen des Rührers wurde die Zeit bis zur vollständigen Phasentrennung ermittelt (siehe Tabelle 2). Das Beispiel zeigt, dass sich ein niedriger Benzolgehalt sowohl in der sauren Wäsche als auch in der neutralen Wäsche positiv auf die Phasentrennzeit auswirkt und damit bereits durch eine teilweise Abtrennung des Benzols eine Verringerung der für die Phasentrennung erforderliche Behältergröße bewirkt werden kann.

Tabelle 2: Ergebnisse aus Beispiel 5.
(a) Versuche zur sauren Wäsche mit Roh-Nitrobenzol enthaltend 2000 ppm an Nitrophenolen

| | | | |
|---|---|---|---|
| Benzolgehalt | 2% | 6% | 9% |
| Phasentrennzeit | 78 s | 212 s | 238 s |

(b) Versuche zur neutralen Wäsche mit gereinigtem Nitrobenzol

| | | | |
|---|---|---|---|
| Benzolgehalt | 0% | 4% | 9% |
| Phasentrennzeit | 11 s | 18 s | 31 s |

## Patentansprüche

1. Verfahren zur kontinuierlichen Herstellung von Nitrobenzol durch
a) Nitrierung von Benzol mit einem Gemisch aus Salpetersäure und Schwefelsäure unter adiabaten Bedingungen;
b) Trennung des in Schritt a) erhaltenen Verfahrensproduktes durch Phasentrennung in eine wässrige Phase, welche mindestens Schwefelsäure, Nitrobenzol und Benzol enthält, und in eine organische Phase, welche mindestens Nitrobenzol und Benzol enthält;
c) Überführung der in Schritt b) erhaltenen wässrigen Phase in einen Verdampfer, in welchem die Schwefelsäure durch Druckerniedrigung aufkonzentriert wird, wobei dem Verdampfer ein gasförmiger Strom, enthaltend mindestens Wasser, Nitrobenzol und Benzol, entnommen und anschließend kondensiert wird, und wobei die erhaltene aufkonzentrierte Schwefelsäure in Schritt a) zurückgeführt wird;
d) destillative Abtrennung von 20 Massen-% bis 100 Massen-% des in der in Schritt b) gewonnenen organischen Phase enthaltenen Benzols durch Verdampfung von Benzol unter Ausnutzung der in Schritt a) angefallenen adiabaten Reaktionswärme in der Verdampfung, wobei ein an Benzol abgereichertes vorgereinigtes Nitrobenzol erhalten wird;
e) Wäsche des in Schritt d) erhaltenen vorgereinigten Nitrobenzols und anschließende Abtrennung von Wasser durch Phasentrennung, wobei gereinigtes Nitrobenzol erhalten wird.

2. Verfahren nach Anspruch 1, bei dem in Schritt d) 20 Massen-% bis 99,8 Massen-% des in der in Schritt b) gewonnenen organischen Phase enthaltenen Benzols abgetrennt werden und sich an Schritt e) anschließt:
f) Destillative Abtrennung von Benzol und Wasser aus dem in Schritt e) erhaltenen gereinigtem Nitrobenzol, wobei getrocknetes Rein-Nitrobenzol erhalten wird.

3. Verfahren nach Anspruch 1, bei dem der in Schritt c) erhaltene kondensierte gasförmige Strom, enthaltend mindestens Wasser, Nitrobenzol und Benzol, nach Abtrennung von Wasser der destillativen Abtrennung von Benzol in Schritt d) zugeführt wird.

4. Verfahren nach Anspruch 1, bei dem die Wäsche in Schritt e) aus mindestens jeweils einer sauren, alkalischen und neutralen Waschstufe besteht.

5. Verfahren nach Anspruch 1, bei dem in Schritt d) > 99,8 Massen-% bis 100 Massen-% des in der in Schritt b) gewonnenen organischen Phase enthaltenen Benzols destillativ abgetrennt werden.

6. Verfahren nach Anspruch 5, bei dem das in Schritt e) gewonnene gereinigte Nitrobenzol nicht weiter aufgereinigt wird.

7. Verfahren nach Anspruch 2, bei dem das in Schritt f) gewonnene getrocknete Rein-Nitrobenzol nicht weiter aufgereinigt wird.

## Claims

1. Process for the continuous production of nitrobenzene by
a) nitration of benzene with a mixture of nitric acid and sulfuric acid under adiabatic conditions;
b) separation of the process product obtained in step a) by phase separation into an aqueous phase, which contains at least sulfuric acid, nitrobenzene and benzene, and an organic phase, which contains at least nitrobenzene and benzene;
c) transfer of the aqueous phase obtained in step b) into an evaporator in which the sulfuric acid is concentrated by pressure reduction, wherein a gaseous stream containing at least water, nitrobenzene and benzene is removed from the evaporator and then condensed, and wherein the resulting concentrated sulfuric acid is fed back into step a);
d) separation by distillation of from 20 % by mass to 100 % by mass of the benzene contained in the organic phase obtained in step b) by evaporation of benzene using the adiabatic heat of reaction obtained in step a) in the evaporation, wherein a pre-purified nitrobenzene depleted of benzene is obtained;
e) washing of the pre-purified nitrobenzene obtained in step d) and subsequent separation of water by phase separation, wherein purified nitrobenzene is obtained.

2. Process according to claim 1, in which in step d) from 20 % by mass to 99.8 % by mass of the benzene contained in the organic phase obtained in step b) are separated off and step e) is followed by:
f) separation by distillation of benzene and water from the purified nitrobenzene obtained in step e), wherein dried pure nitrobenzene is obtained.

3. Process according to claim 1, in which the condensed gaseous stream obtained in step c), containing at least water, nitrobenzene and benzene, is fed, after separation of water, to the separation by distillation of benzene in step d).

4. Process according to claim 1, in which the washing in step e) consists of at least one of each of an acid, alkaline and neutral washing stage.

5. Process according to claim 1, in which in step d) from > 99.8 % by mass to 100 % by mass of the benzene contained in the organic phase obtained in step b) are separated off by distillation.

6. Process according to claim 5, in which the purified nitrobenzene obtained in step e) is not purified further.

7. Process according to claim 2, in which the dried pure nitrobenzene obtained in step f) is not purified further.

## Revendications

1. Procédé pour la production continue de nitrobenzène par
a) nitration de benzène avec un mélange d'acide nitrique et d'acide sulfurique dans des conditions adiabatiques ;
b) séparation du produit du procédé, obtenu dans l'étape a), par séparation de phases en une phase aqueuse qui contient au moins de l'acide sulfurique, du nitrobenzène et du benzène, et en une phase organique qui contient au moins du nitrobenzène et du benzène ;
c) transfert de la phase aqueuse, obtenue dans l'étape b), dans un évaporateur dans lequel l'acide sulfurique est concentré par abaissement de la pression, un courant gazeux, contenant au moins de l'eau, du nitrobenzène et du benzène, étant évacué de l'évaporateur et ensuite condensé, et l'acide sulfurique concentré obtenu étant renvoyé dans l'étape a) ;
d) séparation par distillation de 20 % en masse à 100 % en masse du benzène contenu dans la phase organique obtenue dans l'étape b), par évaporation du benzène avec utilisation dans l'évaporation de la chaleur de réaction adiabatique produite dans l'étape a), de sorte qu'on obtient un nitrobenzène pré-purifié, appauvri en benzène ;
e) lavage du nitrobenzène pré-purifié, obtenu dans l'étape d), et séparation subséquente d'eau par séparation de phases, de sorte qu'on obtient du nitrobenzène purifié.

2. Procédé selon la revendication 1, dans lequel dans l'étape d) sont séparés 20 % en masse à 99,8 % en masse du benzène contenu dans la phase organique obtenue dans l'étape b) et à l'étape e) fait suite :
f) séparation par distillation de benzène et d'eau à partir du nitrobenzène purifié obtenu dans l'étape e), de sorte qu'on obtient du nitrobenzène pur séché.

3. Procédé selon la revendication 1, dans lequel le courant gazeux condensé obtenu dans l'étape c), contenant au moins de l'eau, du nitrobenzène et du benzène, est envoyé après séparation de l'eau à la séparation par distillation du benzène dans l'étape d).

4. Procédé selon la revendication 1, dans lequel le lavage dans l'étape e) consiste en au moins, respectivement, un stade de lavage acide, alcalin et neutre.

5. Procédé selon la revendication 1, dans lequel dans l'étape d) sont séparés par distillation > 99,8 % en masse à 100 % en masse du benzène contenu dans la phase organique obtenue dans l'étape b).

6. Procédé selon la revendication 5, dans lequel le nitrobenzène purifié obtenu dans l'étape e) n'est pas purifié davantage.

7. Procédé selon la revendication 2, dans lequel le nitrobenzène pur séché, obtenu dans l'étape f), n'est pas purifié davantage.
